Europäisches Patentamt

European Patent Office

Office européen des brevets

(19)

(11) **EP 1 396 256 A2**

(12) ## DEMANDE DE BREVET EUROPEEN

(43) Date de publication:
**10.03.2004 Bulletin 2004/11**

(51) Int Cl.⁷: **A61K 7/02**, A61K 7/032

(21) Numéro de dépôt: **03292164.5**

(22) Date de dépôt: **03.09.2003**

| (84) Etats contractants désignés:<br>**AT BE BG CH CY CZ DE DK EE ES FI FR GB GR**<br>**HU IE IT LI LU MC NL PT RO SE SI SK TR**<br>Etats d'extension désignés:<br>**AL LT LV MK** | (72) Inventeurs:<br>• **Pays, Karl**<br>  **94550 Chevilly-Larue (FR)**<br>• **Bichon, Yohann**<br>  **75013 Paris (FR)**<br>• **Olivier, Sandrine**<br>  **94240 L'Hay-Les-Roses (FR)** |
|---|---|
| (30) Priorité: **06.09.2002 FR 0211101** | |
| (71) Demandeur: **L'OREAL**<br>**75008 Paris (FR)** | (74) Mandataire: **Le Coupanec, Pascale**<br>**Nony & Associés,**<br>**3 rue de Penthièvre**<br>**75008 Paris (FR)** |

(54) **Composition cosmétique de maquillage pour les fibres kératiniques**

(57)     La présente invention concerne une composition cosmétique de maquillage de fibres kératiniques comprenant au moins une dispersion aqueuse de particules de cire(s) et possédant une teneur en matière sèche supérieure ou égale à 48 % en poids et un module de rigidité plateau Gp supérieur ou égal à 5500 Pa et inférieur à 60 000 Pa.

**Description**

La présente invention se rapporte au maquillage des fibres kératiniques, comme les cils, sourcils et cheveux, et plus particulièrement, au maquillage des cils.

Les compositions de maquillage pour les yeux, encore appelées « mascara » pour les cils et ligneurs ou « eye-liner » pour les paupières, sont généralement constituées d'une cire ou mélange de cires dispersée(s) à l'aide d'au moins un tensioactif dans une phase aqueuse contenant, par ailleurs, des polymères hydrosolubles et des pigments.

C'est généralement à travers le choix qualitatif et quantitatif des cires et polymères que sont ajustées les spécificités d'application recherchées pour les compositions de maquillage, comme par exemple leur fluidité, leur pouvoir couvrant et/ou leur pouvoir recourbant. Ainsi, il est possible de réaliser diverses compositions qui, appliquées notamment sur les cils, induisent des effets variés de type allongement, recourbement et/ou épaississement (effet chargeant).

La présente invention vise plus particulièrement à proposer une composition utile pour réaliser un maquillage épais des fibres kératiniques et notamment des cils, dit encore maquillage chargeant. Au sens de la présente invention, le terme fibres kératiniques couvre les cheveux, les cils et les sourcils et s'étend également aux postiches et faux-cils synthétiques.

Avec les compositions de maquillage actuellement disponibles, cet effet est généralement obtenu par superposition de plusieurs couches de la composition de maquillage au niveau des fibres kératiniques et plus particulièrement des cils. Par ailleurs, dans le cas particulier des cils, cet effet est très souvent associé à une agglomération de plusieurs cils entre eux, c'est-à-dire à une non individualisation des cils.

Pour des raisons évidentes, il serait avantageux d'obtenir cet effet épaississement en une unique application et tout en obtenant une bonne séparation des cils.

Pour ce faire, il serait particulièrement intéressant de disposer d'une composition de maquillage qui soit suffisamment concentrée en matière sèche pour charger significativement le cil dès sa première mise en contact avec ladite composition et qui permette par ailleurs de gainer chaque cil de manière séparée.

Classiquement, les compositions de maquillage classiques pour les yeux possèdent une matière sèche comprise entre 10 % et 40 % en poids. Si l'on cherche à augmenter cette matière sèche au-delà de cette valeur, on se heurte rapidement à un problème de défaut de fluidité. La composition de maquillage devient trop épaisse à l'application et ne présente plus la déformabilité nécessaire à son application homogène sur toute la surface des cils.

D'une manière inattendue, les inventeurs ont mis en évidence qu'il était possible d'augmenter significativement le taux en matière sèche d'une composition de maquillage pour fibres kératiniques et plus particulièrement sa teneur en cire(s), tout en lui conservant des propriétés rhéologiques satisfaisantes notamment en matière de déformabilité et de consistance au repos, sous réserve du choix d'un système émulsionnant spécifique.

Avantageusement, les compositions revendiquées, bien que présentant une quantité en matière sèche accrue comparativement aux compositions conventionnelles, conservent un module de rigidité plateau Gp approprié à la déformation requise pour leur application homogène par une brosse ou un peigne à la surface des fibres kératiniques et notamment des cils.

L'invention a ainsi pour objet, selon l'un de ses aspects, une composition de maquillage des fibres kératiniques, caractérisée en ce qu'elle comprend au moins une dispersion aqueuse de particules de cire(s) et en ce qu'elle possède une teneur en matière sèche supérieure ou égale à 48 % en poids et un module de rigidité plateau Gp supérieur ou égal à 5500 Pa et inférieur à 60 000 Pa.

La présente invention vise également un procédé de maquillage des fibres kératiniques, caractérisé par le fait que l'on applique sur lesdites fibres une composition conforme à l'invention.

Elle se rapporte en outre à l'utilisation d'une composition conforme à l'invention pour obtenir un maquillage chargeant, et le cas échéant, séparant des fibres kératiniques et notamment des cils et des sourcils.

Au sens de la présente invention, on entend qualifier par le terme « chargeant » la notion d'un maquillage épais des cils.

De manière inattendue, il a ainsi été constaté qu'il était possible de préparer des compositions de maquillage possédant un module de rigidité élevé, c'est-à-dire supérieur ou égal à 5500 Pa et notamment supérieur ou égal à 7000 Pa et une teneur en matière sèche supérieure ou égale à 48 % en poids, grâce à la mise en oeuvre, dans ces compositions, d'un système d'émulsionnant spécifique.

En particulier, la composition selon l'invention possède un module de rigidité plateau inférieur à 60 000 Pa, de manière à lui conférer une déformabilité suffisante pour son application sur la surface à maquiller.

<u>CARACTERISATION DE LA TENEUR EN MATIERE SECHE</u>

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec» des compositions selon l'invention, est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :

On étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \times (\text{masse sèche} / \text{masse humide}).$$

Les compositions selon l'invention se caractérisent par une teneur en matière sèche supérieure ou égale à 48 %, en poids.

Les compositions selon l'invention se caractérisent également par une teneur en matière sèche inférieure ou égale à 85 % en poids, notamment inférieure ou égale à 75 % en poids et en particulier inférieure ou égale à 65 % en poids.

CARACTERISATIONS RHEOLOGIQUES

Les compositions conformes à l'invention sont caractérisées par un comportement viscoélastique.

De façon générale, un matériau est dit viscoélastique quand, sous l'effet du cisaillement, il possède à la fois les caractéristiques d'un matériau purement élastique, c'est à dire capable de stocker de l'énergie et les caractéristiques d'un matériau purement visqueux, c'est à dire capable de dissiper de l'énergie.

Plus particulièrement, le comportement viscoélastique des compositions conformes à l'invention peut être caractérisé par son module de rigidité G, son élasticité δ et son seuil d'écoulement $\tau_c$ ; ces paramètres sont notamment définis dans l'ouvrage "Initiation à la rhéologie", G. Couarraze et J.L. Grossiord, 2$^{\text{ème}}$ édition, 1991, Edition Lavoisier-Tec 1 Doc.

Ces paramètres sont déterminés par des mesures effectuées à 25 °C ± 0,5 °C à l'aide du rhéomètre à contrainte imposée Haake RheoStress 600® de la société ThermoRhéo, équipé d'un mobile en acier inoxydable à géométrie plan/plan, le plan ayant un diamètre de 20 mm et un entrefer (distance entre le plan inférieur - appelé plan stator - sur lequel est déposée la composition et le plan supérieur - appelé plan rotor) de 0,3 mm. Les 2 plans sont striés pour limiter les phénomènes de glissement aux parois des plans.

Les mesures dynamiques sont réalisées en appliquant une variation harmonique de la contrainte. Dans ces expériences, les amplitudes du cisaillement, de la vitesse de cisaillement et de la contrainte sont faibles de manière à rester dans les limites du domaine viscoélastique linéaire du matériau (conditions permettant d'évaluer les caractéristiques rhéologiques de la composition au repos).

Le domaine linéaire viscoélastique est généralement défini par le fait que la réponse du matériau (i.e. la déformation) est à tout moment directement proportionnelle à la valeur de la force appliquée (i.e. la contrainte). Dans ce domaine, les contraintes appliquées sont faibles et le matériau subit des déformations sans modifier sa structure microscopique. Dans ces conditions, le matériau est étudié « au repos » et de façon non destructive.

La composition est soumise à un cisaillement harmonique selon une contrainte τ(t) variant de façon sinusoïdale selon une pulsation ω (ω = 2Πν, ν étant la fréquence du cisaillement appliqué). La composition ainsi cisaillée subie une contrainte τ(t) et répond selon une déformation γ(t) correspondant à des micro déformations pour lesquelles le module de rigidité varie peu en fonction de la contrainte imposée.

La contrainte τ(t) et la déformation γ(t) sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. $\delta$ est l'angle de déphasage entre la contrainte et la déformation.

Les mesures sont effectuées à une fréquence de 1 Hz (v= 1 Hz).

On mesure ainsi l'évolution du module de rigidité G (correspondant au rapport de $\tau_o$ sur $\gamma_o$) et de l'élasticité $\delta$ (correspondant à l'angle de déphasage de la contrainte appliquée par rapport à la déformation mesurée) en fonction de la contrainte $\tau(t)$ appliquée.

On mesure en particulier la déformation de la composition pour la zone de contrainte dans laquelle la variation du module de rigidité G et de l'élasticité $\delta$ est inférieure à 7 % (zone des microdéformations) et on détermine ainsi les paramètres dits « plateaux » Gp et $\delta_p$. La contrainte seuil $\tau_c$ (correspondant à la force minimale qu'il est nécessaire d'appliquer à la composition pour provoquer son écoulement) est déterminée à partir de la courbe $\delta = f(\tau)$ et correspond à la valeur de $\tau$ pour laquelle $\delta (\tau_c) = 1,05 \delta_p$.

Le comportement viscoélastique des compositions selon l'invention est notamment caractérisé par un module de rigidité plateau Gp supérieur ou égal à 5500 Pa, et en particulier qui peut être supérieur ou égal à 7000 Pa, voire supérieur ou égal à 10 000 Pa.

En particulier, les compositions selon l'invention possèdent un module de rigidité plateau Gp inférieur à 60 000 Pa, voire inférieur à 50 000 Pa, notamment inférieur à 40 000 Pa et en particulier inférieur à 30 000 Pa.

En outre, les compositions selon l'invention peuvent présenter une élasticité plateau $\delta_p$ pouvant aller de 1° à 45°, et mieux allant de 5° à 30°.

Les compositions conformes à l'invention peuvent par ailleurs posséder un seuil d'écoulement $\tau_c$ allant de 5 Pa à 3500 Pa, et de préférence allant de 20 Pa à 1000 Pa, ce qui signifie que la composition selon l'invention ne s'écoule pas sous son propre poids mais qu'il est nécessaire d'appliquer à la composition une contrainte minimale pour provoquer son écoulement.

CIRE

Les compositions selon l'invention comprennent, au moins une dispersion aqueuse de particules d'une cire ou d'un mélange de cires.

Au sens de la présente invention, la cire ou le mélange de cires présent dans la composition selon l'invention sera désignée sous le terme général « phase cireuse ».

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur à 45° environ, et en particulier supérieur à 55 °C.

Le point de fusion de la cire peut être mesuré à l'aide d'un calorimètre à balayage différentiel (D.S.C.), par exemple le calorimètre vendu sous la dénomination DSC 30 par la société METLER.

Le protocole de mesure est le suivant :

Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides et rigides à température ambiante, d'origine animale, végétale, minérale ou de synthèse et leurs mélanges.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Désert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut encore citer les cires de silicone, les cires fluorées.

On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A-2 792 190.

La composition selon l'invention contient généralement de 0,1 à 40 % en poids de cire(s), en particulier elle peut en contenir de 5 à 40 %, plus particulièrement de 20 à 40 % et mieux de 30 à 40 % en poids.

La cire ou le mélange de cires est présent, dans les compositions selon l'invention, notamment sous la forme d'une dispersion aqueuse de particules dont la taille exprimée en diamètre « effectif » moyen en volume D[4,3] tel que défini ci-après, peut être avantageusement inférieure ou égale à 1 μm.

Les particules de cire peuvent présenter des formes variées. Elles peuvent notamment être sphériques.

CARACTERISATION DES TAILLES DE PARTICULES

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

Les particules de cire(s) de la phase cireuse dans les compositions selon l'invention peuvent être caractérisées par une taille exprimée en diamètre « effectif » moyen en volume D[4,3] inférieure ou égale à 1 µm, notamment inférieure ou égale à 0,75 µm et mieux inférieure ou égale à 0,55 µm.

La taille des particules est liée principalement à la nature du système émulsionnant utilisé pour réaliser la dispersion.

SYSTEME EMULSIONNANT

Selon l'invention, on utilise généralement un émulsionnant choisi de manière appropriée pour l'obtention d'une émulsion huile-dans-eau. En particulier, on utilise un émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Les compositions selon l'invention peuvent notamment contenir des agents tensioactifs émulsionnants présents notamment en une proportion allant de 0,1 à 40 %, et mieux de 0,5 % à 20 % en poids par rapport au poids total de la composition.

Ces agents tensioactifs peuvent être choisis parmi des agents tensioactifs non ioniques, anioniques, cationiques, amphotériques ou encore des émulsionnants tensioactifs. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p.347-377 de cette référence, pour les tensioactifs anioniques, amphotériques et non ioniques.

Dans le cadre de la présente invention, ce système émulsionnant peut comprendre, au moins un tensioactif ionique et/ou un tensioactif non ionique ayant un HLB supérieur ou égal à 8 à 25 °C ou au moins l'un de ces deux tensioactifs associé à au moins un polymère gélifiant.

TENSIOACTIF NON IONIQUE DE HLB SUPERIEUR OU EGAL A 8

A titre représentatif et non limitatif des agents tensioactifs non ioniques ayant un HLB supérieur ou égal à 8 et pouvant être utilisés seuls ou en mélange dans les compositions de maquillage selon l'invention, on peut citer notamment :

- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination «NEODOL 25-7 »® par SHELL CHEMICALS) ;
- les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P par la société ICI UNIQUEMA;
- les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination « Simulsol 220 TM » par la société SEPPIC ; le stéarate de glycéryle polyéthoxylé à 30 groupes d'oxyde d'éthylène comme le produit TAGAT S vendu par la société GOLDSCHMIDT, l'oléate de glycéryle polyéthoxylé à 30 groupes d'oxyde d'éthylène comme le produit TAGAT O vendu par la société GOLDSCHMIDT, le cocoate de glycéryle polyéthoxylé à 30 groupes d'oxyde d'éthylène comme le produit VARIONIC LI 13 vendu par la société SHEREX, l'isostéarate de glycéryle polyéthoxylé à 30 groupes d'oxyde d'éthylène comme le produit TAGAT L vendu par la société GOLDSCHMIDT et le laurate de glycéryle polyéthoxylé à 30 groupes d'oxyde d'éthylène comme le produit TAGAT I de la société GOLDSCHMIDT.
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination « Tween 60 » par la société UNIQUEMA ;
- la diméthicone copolyol, telle que celle vendue sous la dénomination « Q2-5220 » par la société DOW CORNING,
- la diméthicone copolyol benzoate ( FINSOLV SLB 101 et 201 de la société FINTEX),
- les copolymères d'oxyde propylène et d'oxyde d'éthylène, également appelés polycondensats OE/OP,
- et leurs mélanges.

Les polycondensats OE/OP sont plus particulièrement de copolymères consistant en des blocs polyéthylène glycol et polypropylène glycol, comme par exemple les polycondensats tribloc polyéthylène glycol / polypropylène glycol / polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/1 en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations "SYNPERONIC" comme les "SYNPERONIC PE/ L44" et "SYNPERONIC PE/F127 " par la société ICI.

A cet agent tensioactif non ionique de HLB supérieur ou égal à 8, il peut le cas échéant être associé un ou plusieurs agents tensioactif non ioniques de HLB inférieur à 8 à 25°C.

A titre représentatif et non limitatif de ces agents ayant un HLB inférieur à 8 à 25 °C, on peut plus particulièrement citer :

- les esters et éthers d'oses tels que les stéarate de sucrose, cocoate de sucrose, stéarate de sorbitan et leurs mélanges comme l'Arlatone 2121 commercialisé par la société ICI ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M par la société GOLDSCHMIDT, laurate de glycéryle tel que le produit vendu sous la dénomination IMWITOR 312 par la société HULS, stéarate de polyglycéryl-2, tristéarate de sorbitan, ricinoléate de glycéryle ;
- le mélange de cyclométhicone/diméthicone copolyol vendu sous la dénomination "Q2-3225C" par la société DOW CORNING.

La quantité en tensioactif non ionique est généralement ajustée de manière à obtenir une composition possédant les paramètres tels que définis précédemment. La détermination de cette quantité relève des compétences de l'homme de l'art.

A titre illustratif et non limitatif du domaine de l'invention, cette quantité de tensioactif non ionique de HLB supérieur ou égal à 8 peut aller de 0,01 à 40 % en poids, en particulier de 0,1 à 20 % voire de 0,5 à 15 %, et mieux de 0,5 à 10 % en poids.

TENSIOACTIF IONIQUE

D'une manière générale, la composition revendiquée contient un tensioactif ionique notamment en association avec au moins un tensioactif non ionique de HLB supérieur ou égal à 8 et/ou au moins un polymère gélifiant.

Les tensioactifs ioniques mis en oeuvre dans le cadre de la présente invention peuvent être anioniques ou cationiques. Toutefois, généralement, le choix d'au moins un tensioactif anionique est privilégié.

A titre représentatif des tensioactifs anioniques convenant à l'invention, on peut plus particulièrement citer :

- les sels d'acides gras en $C_{16}$-$C_{30}$ notamment ceux dérivant des amines, comme le stéarate de triéthanolamine;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ;
- les sulfosuccinates tels que le "Disodium PEG-5 citrate lauryl sulfosuccinate" et le "Disodium ricinoleamido MEA sulfosuccinate"
- les alkyléthersulfates tels que le lauryl éther sulfate de sodium ;
- les iséthionates ;
- les acylglutamates tels que le "Disodium hydrogenated tallow glutamate" (AMISOFT HS-21 R commercialisé par la société AJINOMOTO) et leurs mélanges.

Convient tout particulièrement à l'invention, le stéarate de triéthanolamine. Ce dernier est généralement obtenu par simple mélange de l'acide stéarique et de la triéthanolamine.

A titre représentatif des tensioactifs cationiques, on peut notamment citer :

- les alkyl-imidazolidinium tels que l'étho-sulfate d'isostéaryléthylimidonium,
- les sels d'ammonium tels que le chlorure de N,N,N-triméthyl-1-docosanaminium (chlorure de Behentrimonium).

Les compositions selon l'invention peuvent également contenir un ou plusieurs tensioactifs amphotériques comme les N-acyl-aminoacides tels que les N-alkylaminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination « PECOSIL PS 100 » par la société PHOENIX CHEMICAL.

D'une manière générale, les compositions selon l'invention peuvent contenir de 0,01 à 30 % en poids, en particulier de 0,1 à 15 % en poids voire de 0,5 à 10 % en poids de tensioactif ionique.

POLYMERE GELIFIANT

Les compositions selon l'invention peuvent contenir en outre au moins un polymère gélifiant.

Selon la présente invention, on entend par "polymère gélifiant", un polymère apte à gélifier la phase continue, généralement aqueuse, des compositions selon l'invention.

Le polymère gélifiant utilisable selon l'invention, peut notamment être caractérisé par sa capacité à former dans l'eau, au-delà d'une certaine concentration C*, un gel caractérisé par rhéologie oscillatoire ($\upsilon$ = 1 Hz) par un seuil d'écoulement $\tau_c$ au moins égal à 10 Pa. Cette concentration peut varier largement selon la nature de l'agent gélifiant considéré.

A titre illustratif cette concentration est comprise entre 1 et 2 % en poids pour un copolymère acrylamide/acrylamido-2-méthyl propane sulfonate de sodium en émulsion inverse à 40 % dans polysorbate 80/I-C16 comme celui commercialisé sous la dénomination « Simulgel 600 » par la société SEPPIC, et est d'environ 0,5 % en poids pour un copolymère AMPS/méthacrylate de cétéaryle éthoxylé (250E) réticulé par le triméthylolpropane triacrylate (TMPA).

Le polymère gélifiant peut être un polymère hydrosoluble et est donc présent dans la phase aqueuse de la composition sous forme solubilisée.

Plus particulièrement, ce polymère gélifiant peut être choisi parmi :

- les homo- ou copolymères d'acides acrylique ou méthacrylique ou leurs sels et leurs esters et en particulier les produits vendus sous les dénominations «VERSICOL F »ou « VERSICOL K» par la société ALLIED COLLOID, « UTRAHOLD 8 » par la société CIBA-GEIGY, les acides polyacryliques de type SYNTHALEN K,
- les copolymères d'acide acrylique et d'acrylamide vendus sous la forme de leur sel de sodium sous les dénominations « RETEN » par la société HERCULES, le polyméthacrylate de sodium vendu sous la dénomination « DARVAN N°7 » par la société VANDERBILT, les sels de sodium d'acides polyhydroxycarboxyliques vendus sous la dénomination « HYDAGEN F » par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL ou SIMULGEL commercialisés par la société SEPPIC, et
- les copolymères AMPS/méthacrylates d'alkyle polyoxyéthylénés (réticulés ou non), et leurs mélanges. Comme autres exemples de polymères gélifiants hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :

  - les gommes arabiques, la gomme de guar, les dérivés du xanthane, la gomme de karaya ;
  - les alginates et les carraghénanes ;
  - les glycoaminoglycanes, l'acide hyaluronique et ses dérivés ;
  - la résine shellac, la gomme de sandaraque, les dammars, les élémis, les copals ;

- l'acide désoxyribonucléïque ;
- les muccopolysaccharides tels que l'acide hyaluronique, les chondroïtines sulfate, et leurs mélanges.

Le polymère gélifiant est généralement présent dans la composition à une quantité suffisante pour ajuster le module de rigidité à une valeur supérieure à 5500 Pa, voire supérieure à 7000 Pa.

En l'occurrence, le polymère gélifiant peut être présent dans la composition selon l'invention en une teneur en matières sèches allant de 0,1 % à 60 % en poids, de préférence de 0,5 % à 40 % en poids, mieux de 1 % à 30 % en poids, voire de 5 à 20 % en poids par rapport au poids total de la composition.

Il est entendu que cette quantité est par ailleurs susceptible de varier selon que ledit polymère est associé ou non à un tensioactif ionique et/ou non ionique et/ou un agent filmogène qui eux aussi sont susceptible d'agir sur la consistance de ladite composition.

POLYMERE FILMOGENE

La composition selon l'invention peut en outre comprendre un agent filmogène.

Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges.

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés $\alpha,\beta$-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styréniques comme le styrène et l'alpha-méthyl styrène.

Parmi les polycondensats filmogènes, on peut citer les polyuréthanes, les polyesters, les polyesters amides, les polyamides, et les polyurées.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER ; les sulfopolyesters vendus sous le nom de marque Eastman AQ par la société Eastman Chemical Products.

La composition selon l'invention peut comprendre également un agent auxiliaire de filmification favorisant la formation d'un film avec le polymère filmogène.

MILIEU PHYSIOLOGIQUEMENT ACCEPTABLE

Généralement, les compositions selon l'invention sont à base d'eau ou d'un milieu aqueux, c'est-à-dire un mélange d'eau avec au moins un solvant organique.

Le milieu aqueux de la composition peut ainsi comprendre un mélange d'eau et de solvant organique miscible à l'eau comme les monoalcools inférieurs ayant de 1 à 5 atomes de carbone tels que l'éthanol, l'isopropanol, les glycols ayant de 2 à 8 atomes de carbone tels que la glycérine, le propylène glycol, l'éthylène glycol, le 1,3-butylène glycol, le dipropylène glycol, les cétones en $C_3$-$C_4$, les aldéhydes en $C_2$-$C_4$. Le milieu aqueux (eau et éventuellement le solvant organique miscible à l'eau) peut représenter, en pratique, de 5 % à 90 % en poids, par rapport au poids total de la composition.

ADDITIFS

Les compositions revendiquées peuvent également contenir des ingrédients couramment utilisés dans le domaine du maquillage des fibres kératiniques.

La composition selon l'invention peut notamment comprendre une ou plusieurs huiles.

L'huile peut être choisie parmi les huiles volatiles et/ou les huile non volatiles, et leurs mélanges. La composition comprend avantageusement au moins une huile volatile.

Par "huile volatile", on entend au sens de l'invention une huile susceptible de s'évaporer au contact de la peau ou de la fibre kératinique en moins d'une heure, à température ambiante et pression atmosphérique.

Le ou les solvants organiques volatils et les huiles volatiles de l'invention sont des solvants organiques et des huiles cosmétiques volatiles, liquides à température ambiante, ayant une pression de vapeur non nulle à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg). Par « huile non volatile », on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité $\leq 8$ centistokes (8 $10^{-6}$ m$^2$/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

L'huile volatile peut être présente dans la composition selon l'invention en une teneur allant de 0,1 % à 60 % en poids, par rapport au poids total de la composition, de préférence de 0,1% à 30% en poids.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;

- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R5 + R6 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0,1 à 20 % en poids, de préférence de 0,1 % à 12 % en poids, par rapport au poids total de la composition.

La composition peut en outre comprendre d'autres ingrédients habituellement utilisés en cosmétique. De tels ingrédients peuvent être notamment, des agents de coalescence, des charges, des matières colorantes comme les pigments, les nacres ou les colorants liposolubles ou hydrosolubles, les colorants goniochromatiques, des conservateurs, des huiles, des agents hydratants, des parfums, et leurs mélanges, qui sont bien connus de l'état de la technique.

Les pigments peuvent être blancs ou colorés, minéraux et/ou organiques ; ils sont insolubles dans le milieu physiologiquement acceptable de la composition. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome, le bleu ferrique et leurs mélanges. Parmi les pigments organiques, on peut citer le noir de carbone, les pigments de type D & C, et les laques à base de carmin de cochenille, de baryum, strontium, calcium, aluminium et leurs mélanges. On peut aussi utiliser les pigments à effet optique particulier comme les particules de verre recouvertes de métal, notamment d'or, d'argent et de platine.

Les nacres ou pigments nacrés sont des particules irisées, notamment produites par certains mollusques dans leur coquille ou bien synthétisées, insolubles dans le milieu physiologiquement acceptable de la composition. Ils peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth et leurs mélanges. On peut également utiliser les pigments interférentiels, notamment à cristaux liquides ou multicouches.

Les matières colorantes peuvent représenter de 0,01 à 30 %, de préférence de 0,1 à 25 % du poids total de la composition et mieux de 1 à 20 %.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® (Orgasol de chez Atochem), de poly-β-alanine et de polyéthylène, les poudres de polymères de tétrafluoroéthylène comme le Téflon®, la lauroyl-lysine, l'amidon, le nitrure de bore, les micro sphères creuses polymériques telles que celles de chlorure de polyvinylidène/acrylonitrile comme l'ExpanceL® (Nobel Industrie), les poudres acryliques telles que le polytrap® (Dow Corning), les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (SILICA BEADS® de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent représenter de 0,1 à 25 %, et mieux de 1 à 20 % en poids du poids total de la composition.

La composition selon l'invention peut en outre comprendre des fibres qui peuvent permettre une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, notamment de 5 à 500, et en particulier de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, de préférence de 0,1 mm à 5 mm et mieux de 1 mm à 3,5 mm. Leur section peut être comprise dans un cercle de diamètre allant de 2 nm à 500 μm, de préférence allant de 100 nm à 100 μm et mieux de 1 μm à 50 μm. Le poids ou titre des fibres est souvent donné en denier ou décitex et représente le poids en gramme pour 9 km de fil. En particulier, les fibres peuvent avoir un titre choisi dans la gamme allant de 0,15 à 30 deniers et mieux de 0,18 à 18 deniers.

Les fibres peuvent être celles utilisées dans la fabrication des textiles et notamment des fibres de soie, de coton, de laine, de lin, des fibres de cellulose - par exemple extraites notamment du bois, des légumes ou des algues -, de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson).

Par ailleurs, les fibres peuvent être traités ou non en surface, enrobées ou non.

En particulier, on utilise des fibres d'origine synthétiques et en particulier des fibres organiques, comme celles utilisées en chirurgie. Avantageusement, on peut utiliser des fibres insolubles dans l'eau.

Les fibres utilisables dans la composition selon l'invention peuvent être en particulier des fibres de polyamide, de cellulose, de poly-p-phénylène téréphtalamide ou de de polyéthylène. Leur longueur (L) peut aller de 0,1 mm à 5 mm, notamment de 0,25 mm à 1,6 mm et leur diamètre moyen peut aller de 1 μm à 50 μm. En particulier, on peut utiliser les fibres de polyamide commercialisées par les Etablissements P. Bonté sous le nom de "Polyamide 0.9 Dtex 3 mm", ayant un diamètre moyen de 6 μm, un titre d'environ 0.9 dtex et une longueur allant de 0,3 mm à 5 mm. On peut aussi utiliser les fibres de celluloses (ou de rayonne) ayant un diamètre moyen de 50 μm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

La composition selon l'invention peut également comprendre des fibres dites "rigides", par opposition aux fibres citées précédemment, qui ne sont pas des fibres rigides.

Les fibres rigides, initialement sensiblement droites, lorsqu'elles sont placées dans un milieu dispersant, ne voient pas leur forme sensiblement modifiée.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :

- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;
- de polyamide, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;
- de polyimide-amide telles que celles vendues sous les dénomination "KERMEL", " KERMEL TECH" par la société RHODIA ;
- de poly-(p-phénylène-téréphtalamide) (ou d'aramide) notamment vendues sous la dénomination Kevlar ® par la société DUPONT DE NEMOURS ;

- des fibres à structure multicouche comprenant des couches alternées de polymères choisis parmi les polyesters, les polymères acryliques, les polyamides, telles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Des fibres rigides particulièrement avantageuses sont les fibres de polymide-amide aromatiques.

Des fils ou fibres de polyimide-amide, pouvant être utilisés pour les compositions selon l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auxquels on pourra se référer.

En particulier, les fibres de polyimide-amide aromatique peuvent être des fibres de polyimide-amide comprenant des motifs répétitif de formule :

obtenu par polycondensation du toluylène diisocyanate et de l'anhydride triméllitique.

Les fibres peuvent êtres présentes dans la composition selon l'invention en une teneur allant de 0,01 à 10 % en poids, notamment de 0,05 à 5 % en poids par rapport au poids total de la composition.

Bien entendu, l'homme du métier veillera à choisir ce ou ces ingrédients et/ou leur quantité, de manière telle que les propriétés avantageuses de la composition selon l'invention ne soient pas, ou substantiellement pas, altérées par l'adjonction envisagée.

Les compositions selon l'invention peuvent aussi contenir un ou plusieurs adjuvants usuels tels que des parfums, des agents conservateurs, des agents alcalinisants ou acidifiants, des agents de textures, des additifs d'étalement, des plastifiants ainsi que des ingrédients actifs hydrosolubles utilisés habituellement dans les préparations cosmétiques pour les fibres kératiniques.

PROCEDE DE PREPARATION

Les compositions selon l'invention sont généralement obtenues de manière standard par formation à chaud d'une émulsion.

Plus précisément, ces compositions sont obtenues par chauffage de la cire et le ou les tensioactif(s) considéré (s) de HLB ≤ 8 à une température supérieure à la température de fusion de la cire et non supérieure à 100 °C, jusqu'à fusion complète de la cire, puis ajout progressivement de l'eau, et le cas échéant le polymère gélifiant et/ou des tensioactifs de HLB > 8, portée à une température au moins égale à la température précédente, en agitant jusqu'à retour à la température ambiante.

Les ingrédients liposolubles, par exemple des céramides, sont généralement ajoutés à la cire avant la réalisation de l'émulsion.

Des ingrédients hydrosolubles peuvent être ajoutés dans l'eau utilisée pour réaliser l'émulsion, ou dans l'émulsion finalement obtenue.

De même, des ingrédients secondaires éventuellement présents dans la composition sont ajoutés selon les cas soit dans les produits de départ, soit dans la composition terminée.

Les compositions de l'invention peuvent être appliquées sur les cils, à l'aide d'une brosse ou d'un peigne.

L'effet épaississant recherché dans le cadre de la présente invention peut par ailleurs être renforcé en sélectionnant tout particulièrement le dispositif pour l'application de ladite composition.

En l'occurrence, il est particulièrement avantageux de procéder, dans le cas du maquillage des cils, à l'application de ladite composition avec une brosse de maquillage telle que décrites dans les brevets FR 2 701 198, FR 2 605 505, EP 792 603 et EP 663 161.

Les exemples qui suivent sont présentés à titre illustratif et non limitatif de l'invention.

Les quantités indiquées sont en pourcentage pondéral et exprimées par rapport au poids total de la composition, sauf instructions contraires.

Les mesures de rhéologie ont été effectuées sur un rhéomètre à contrainte imposée Haake RheoStress 600 dans les conditions suivantes :

- température de mesure 25 °C,
- palier de 180 sec à 25 °C avant le début de mesure,
- balayage en contrainte de 1 à 10 000 Pa,
- fréquence de mesure : 1 Hz.

Les polymères gélifiants et tensioactifs utilisés sont les suivants :

- hydroxyéthyl cellulose quaternisé par chlorure de 2,3 époxypropyl triméthyl ammonium commercialisé sous la dénomination «UCARE POLYMER JR 400» par la société AMERCHOL (DOW CHEMICAL),
- hydroxyéthyle cellulose commercialisée sous le nom de « Cellobond HEC 5000 A » distribué par la société BRENNTAG,
- polyméthacrylate de sodium dans l'eau à 25 % non stabilisé commercialisé sous la dénomination « Darvan 7 » par la société VANDERBILT,
- copolymère acrylate d'éthyle/méthacrylate de méthyle (80/20) réticulé, en dispersion aqueuse à 50 % commercialisé sous la dénomination « Daitosol 5000 AD» par la société DAITO,
- copolymère acrylamide/acrylamido-2-méthyl propane sulfonate de sodium en émulsion inverse à 40 % dans polysorbate 80/I-C16 commercialisé sous la dénomination « Simulgel 600 » par la société SEPPIC,
- monostéarate de glycéryle oxyéthyléné (200 OE) commercialisé sous la dénomination « Simulsol » par la société SEPPIC,
- monostéarate de glycéryle oxyéthyléné (30 OE) commercialisé sous la dénomination « Tagat S » par la société DEGUSSA/GOLDSCHMIDT,
- copolymère AMPS/méthacrylate de cétéaryle éthoxylé (25 OE) réticulé par triméthylolpropane triacrylate (TMPTA).

Le stéarate de triéthanolamine est préparé in situ par mélange d'acide stéarique et de triéthanolamine à 99 %.

Les formulations suivantes de mascara ont été préparées :

| Formulation A : | |
|---|---|
| amino-2 méthyl-2 propane di-ol-1,3 | 0,23 |
| cire de Carnauba | 6,26 |
| D-Panthénol | 0,54 |
| oxyde de fer noir (CI:77499) | 3,26 |
| bleu d'outremer (CI:77007) | 4,49 |
| hydroxyéthyl cellulose quaternisée par chlorure de 2,3 époxypropyl triméthyl ammonium | 0,10 |
| hydroxy-éthyl cellulose | 0,95 |
| mélange de polydiméthylsiloxane et de silice hydratée | 0,17 |
| polyméthacrylate de sodium dans l'eau à 25 % non stabilisé | 5,42 |
| gomme arabique ; polysaccharides ; arabinose/galactose/rhamnose/acide glucuronique | 3,67 |
| P-hydroxybenzoate de propyle | 0,22 |
| P-hydroxybenzoate de méthyle | 0,26 |
| polydiméthylsiloxane oxyéthyléné (20 OE) oxypropylène (20 OP)(DP : 170 - viscosité : 1000 CST) | 0,22 |
| copolymère acrylate d'éthyle/méthacrylate de méthyle (80/20) réticulé, en dispersion aqueuse à 50 % | 4,33 |
| monostéarate de glycéryle oxyéthyléné (200 OE) | 3,26 |
| monostéarate de glycéryle oxyéthyléné (30 OE) | 1,09 |
| copolymère AMPS/méthacrylate de cétéaryle éthoxylé(25 OE) réticulé par triméthylolpropane triacrylate(TMPTA) | 1,10 |
| huile de Jojoba (Simmondsia Chinensis) isomérisée TRANS de TF 45°C | 26,33 |
| eau déminéralisée stérilisée | qsp 100% |

La formulation correspondante possède une texture crémeuse et permet d'obtenir un maquillage épais des cils.

| Formulation B : | |
|---|---|
| amino-2 méthyl-2 propane di-ol-1,3 | 0,21 |
| acide stéarique (C16-18:50/50) | 2,49 |
| cire de Carnauba | 3,00 |
| D-Panthénol | 0,50 |
| phenoxy-2 éthanol | 0,50 |
| oxyde de fer noir (CI:77499) | 3,00 |
| bleu d'outremer (CI:77007) | 4,14 |
| hydroxéthyl cellulose quaternisée par chlorure de 2,3 époxypropyl triméthyl ammonium | 0,10 |
| hydroxéthyl cellulose | 0,88 |
| mélange de polydiméthylsiloxane et de silice hydratée | 0,15 |
| poly méthacrylate de sodium dans l'eau à 25 % non stabilise | 5,00 |
| gomme arabique ; polysaccharides ; arabinose/galactose/rhamnose/acide glucuronique | 3,38 |
| cire d'abeille blanche, pure | 6,50 |
| Glycérine | 2,00 |
| P-hydroxybenzoate de propyle | 0,20 |
| P-hydroxybenzoate de méthyle | 0,25 |
| tri-éthanolamine à 99 % | 1,02 |
| polydiméthylsiloxane oxyéthylène (20 OE) oxypropylène (20 OP)(DP : 170 - viscosité : 1000 CST) | 0,20 |
| huile de Jojoba hydrogénée copolymère acrylate d'éthyle/méthacrylate de méthyle (80/20) | 6,27 |
| réticulé, en dispersion aqueuse à 50 % | 4,00 |
| copolymère acrylamide/acrylamido-2-méthyl propane sulfonate de sodium en émulsion inverse à 40 % dans polysorbate 80/I-C16 | 2,00 |
| monostéarate de glycéryle oxyéthyléné (200 OE) | 3,00 |
| huile de Jojoba (Simmondsia Chinensis) isomérisée TRANS de TF 45°C | 6,27 |
| eau déminéralisée stérilisée | qsp 100 % |

La formulation possède une texture crémeuse particulièrement appropriée à l'application par brosse et permet d'obtenir un maquillage épais des cils.

A des fins de clarté, les systèmes émulsionnants et la quantité de cire mis en oeuvre dans chacune des formulations sont précisément identifiés dans le tableau I ci-après.

Les paramètres rhéologiques et la teneur en matière sèche (extrait sec) ont été caractérisés pour chacune des formulations. Ils sont présentés en tableau II.

Tableau I

| | T.A non ionique (%m) | | Stéarate de triéthanolamine (%m) | | Polymère gélifiant (%m) | | Cire (%m) | | |
|---|---|---|---|---|---|---|---|---|---|
| | PEG 30-GS | PEG 200 GS | Acide stéarique | Triéthanolamine 99% | Simulgel 600 | copolymères AMPS/méthacrylates d'alkyle polyoxyéthylénés | Cire de Carnauba | Cire d'abeille | Huile de Jojoba isomérisée, hydrogénée |
| A | 1,09 | 3,26 | — | — | — | 1,10 | 6,26 | — | 26,33 |
| B | — | 3,00 | 2,49 | 1,02 | 2,00 | — | 3,00 | 6,50 | 12,54 |

Tableau II

| | Matière sèches (%m) | Module de rigidité (10³ Pa) | Contrainte Seuil (Pa) | Angle de perte (°) | Taille des particules de cire (µm) |
|---|---|---|---|---|---|
| A | 56,4 | 12 | 40 | 20 | 2,3 |
| B | 49,2 | 20 | 100 | 16 | 0,82 |

**Revendications**

1. Composition cosmétique de maquillage de fibres kératiniques, **caractérisée en ce qu'**elle comprend au moins une dispersion aqueuse de particules de cire(s) et **en ce qu'**elle possède une teneur en matière sèche supérieure ou égale à 48 % en poids et un module de rigidité plateau Gp supérieur ou égal à 5500 Pa et inférieur à 60 000 Pa.

2. Composition selon la revendication 1, **caractérisée en ce que** le module de rigidité plateau Gp est supérieur ou égal à 7000 Pa.

3. Composition selon la revendication 1 ou 2, **caractérisé en ce qu'**elle possède un système émulsionnant possédant à 25 °C une balance HLB, supérieure ou égale à 8.

4.  Composition cosmétique selon la revendication 1, 2 ou 3, **caractérisée en ce qu'**elle comprend au moins un tensioactif ionique.

5.  Composition selon la revendication 4, **caractérisée en ce que** ledit tensioactif ionique est au moins un tensioactif anionique.

6.  Composition selon la revendication 5, **caractérisée en ce que** ledit tensioactif anionique est choisi parmi les sels d'acides gras en $C_{16}$-$C_{30}$ ; les sels d'acides gras polyoxyéthoxylénés, les esters phosphoriques et leurs sels ; les alkyléthersulfates, les sulfosuccinates ; les iséthionates et les acylglutamates et leurs mélanges.

7.  Composition selon la revendication 4, 5 ou 6, **caractérisée en ce que** le tensioactif ionique comprend au moins le stéarate de triéthanolamine.

8.  Composition selon l'une quelconque des revendications 4 à 7, **caractérisée en ce que** ledit tensioactif ionique est présent à raison de 0,01 à 30 % en poids et notamment de 0,1 à 15 % en poids par rapport au poids total de la composition.

9.  Composition selon l'une quelconque des revendications 4 à 8, **caractérisée en ce que** ledit tensioactif ionique est associé à au moins un tensioactif non ionique de HLB supérieur ou égal à 8 à 25 °C et/ou un polymère gélifiant.

10. Composition selon la revendication 9, **caractérisée en ce que** ledit tensioactif non ionique de HLB supérieur ou égal à 8 est choisi parmi les éthers d'alcools gras oxyéthylénés et/ou oxypropylénés, les esters d'acide gras et de polyéthylène glycol, les éthers de glycérol oxyéthylénés et/ou oxypropylénés, les esters d'acide gras et d'éther de sorbitol oxyéthylénés et/ou oxypropylénés et les copolymères d'oxyde propylène et d'oxyde d'éthylène et leurs mélanges.

11. Composition selon la revendication 10, **caractérisée en ce qu'**il est choisi parmi les éthers oxyéthylénés de l'alcool cétéarylique à 30 groupes oxyéthylénés, éther oxyéthyléné d'un mélange d'alcools gras en $C_{12}$-$C_{15}$ comportant 7 groupes oxyéthylénés, stéarate de PEG-50, stéarate de PEG-40, monostéarate de PEG-200 glycéryle, stéarate de glycéryle polyéthoxylé à 30 groupes d'oxyde d'éthylène, oléate de glycéryle polyéthoxylé à 30 groupes d'oxyde d'éthylène, cocoate de glycéryle polyéthoxylé à 30 groupes d'oxyde d'éthylène, isostéarate de glycéryle polyéthoxylé à 30 groupes d'oxyde d'éthylène, laurate de glycéryle polyéthoxylé à 30 groupes d'oxyde d'éthylène, diméthicone copolyol ; dimethicone copolyol benzoate et leurs mélanges.

12. Composition selon l'une quelconque des revendications 9 à 11, **caractérisée en ce qu'**elle comprend au moins un tensioactif non ionique de HLB supérieur ou égal à 8 à raison de 0,01 % à 40 % en poids, en particulier de 0,1 % à 20 % en poids par rapport au poids total de la composition.

13. Composition selon l'une quelconque des revendications précédentes, **caractérisée en ce qu'**elle comprend de 0,1 à 40 % et notamment de 0,5 à 20 % en poids de tensioactifs.

14. Composition selon l'une des revendications 9 à 13, **caractérisée en ce que** le polymère gélifiant est choisi parmi les homo- ou co-polymères d'acides acrylique ou méthacrylique, leurs sels et leurs esters ; les acides polyacryliques ; les sels de copolymères d'acide acrylique et d'acrylamide ; les sels de sodium d'acides polyhydroxycarboxyliques ; les sels de sodium d'acides polyhydroxycarboxyliques ; les copolymères acides polyacryliques/acrylates d'alkyle ; l'AMPS ; les copolymères AMPS/acrylamide ; les copolymères AMPS/méthacrylates d'alkyle polyxyéthylénés (réticulés ou non) ; les protéines d'origine végétale ou animale ; les polymères de chitine ou de chitosane anioniques, cationiques, amphotères ou non-ioniques ; les polymères de cellulose ; les polymères vinyliques ; les polymères d'origine naturelle et leurs mélanges.

15. Composition selon l'une quelconque des revendications 9 à 14, **caractérisée en ce que** le polymère gélifiant est choisi parmi les homo- ou co-polymères d'acides acrylique ou méthacrylique ou leurs sels et leurs esters, les copolymères d'acide acrylique et d'acrylamide, les copolymères d'acide polyacrylique/acrylates d'alkyle, l'AMPS (Acide polyacrylamidométhyl propane sulfonique), les copolymères AMPS/acrylamide et les copolymères AMPS/méthacrylates d'alkyle polyoxyéthylénés et leurs mélanges.

**16.** Composition selon l'une quelconque des revendications 9 à 15, **caractérisée en ce que** ledit polymère est présent à une teneur en matière sèche variant de 0,1 à 60 % et notamment de 0,5 à 40 % en poids par rapport au poids de ladite composition.

**17.** Composition selon l'une quelconque des revendications précédentes, **caractérisée en ce qu'**elle comprend en outre un polymère filmogène.

**18.** Composition selon la revendication 17, **caractérisée en ce que** le polymère filmogène est choisi parmi les polymères synthétiques, de type radicalaire ou de type polycondensat, les polymères d'origine naturelle et leurs mélanges.

**19.** Composition selon l'une quelconque des revendications précédentes, **caractérisée en ce que** la cire est choisie parmi les cires, solides et rigides à température ambiante, d'origine animale ou végétale.

**20.** Composition selon l'une quelconque des revendications précédentes, **caractérisée en ce que** la cire présente un point de fusion supérieur à 45 °C, et en particulier supérieur à 55 °C.

**21.** Composition selon l'une quelconque des revendications précédentes, **caractérisée en ce que** la cire est choisie parmi les cires hydrocarbonées comme la cire d'abeilles, la cire de lanoline, et les cires d'insectes de Chine ; la cire de riz, la cire de Carnauba, la cire de Candellila, la cire d'Ouricurry, la cire d'Alfa, la cire de fibres de liège, la cire de canne à sucre, la cire du Japon et la cire de sumac ; la cire de montan ; les cires microcristallines, les paraffines et l'ozokérites ; les cires de polyéthylène ; les cires obtenues par la synthèse de Fisher-Tropsch ; les copolymères cireux ainsi que leurs esters ; les cires obtenues par hydrogénation catalytique d'huiles animales ou végétales ayant des chaînes grasses, linéaires ou ramifiées en C8-C32 comme l'huile de jojoba hydrogénée, l'huile de jojoba isomérisée, l'huile de tournesol hydrogénée, l'huile de ricin hydrogénée, l'huile de coprah hydrogénée et l'huile de lanoline hydrogénée, le tétrastéarate de di-(triméthylol-1,1;1 propane) et le tétrabéhénate de di-(triméthylol-1,1,1 propane).

**22.** Composition selon l'une quelconque des revendications précédentes, **caractérisée en ce qu'**elle comprend une phase cireuse à raison de 0,1 à 40 % et notamment de 5 à 40 % en poids.

**23.** Composition selon l'une quelconque des revendications précédentes, **caractérisée en ce que** la phase cireuse est présente sous la forme d'une dispersion de particules de cire possédant une taille exprimée en diamètre « effectif » moyen en volume D[4,3] inférieure ou égale à 1 micron et notamment inférieure ou égale à 0,75 micron.

**24.** Composition selon l'une quelconque des revendications précédentes, **caractérisée en ce qu'**elle possède un seuil d'écoulement $\tau_c$ mesuré par rhéologie oscillatoire ($\upsilon$ = 1Hz) variant de 5 à 3500 Pa et notamment de 20 à 1000 Pa.

**25.** Composition selon l'une quelconque des revendications précédentes, **caractérisée en ce qu'**elle présente une élasticité plateau $\delta_p$ variant de 1° à 45°, et notamment allant de 5° à 30°.

**26.** Composition selon l'une quelconque des revendications précédentes, **caractérisée en ce qu'**elle contient de 0,01 % à 30 % en poids d'au moins une matière colorante et/ou de 0,1 à 25 % en poids d'une charge.

**27.** Utilisation d'une composition selon l'une quelconque des revendications 1 à 26 pour obtenir un maquillage chargeant des fibres kératiniques et notamment des cils et des sourcils.

**28.** Procédé de maquillage des fibres kératiniques, **caractérisé en ce que** l'on applique sur lesdites fibres kératiniques notamment les cils une composition telle que définie selon l'une quelconque des revendications 1 à 26.